# EUROPÄISCHE PATENTANMELDUNG

(11) **EP 2 674 208 A1**
(43) Veröffentlichungstag der Anmeldung: **18.12.2013**
(21) Anmeldenummer: 12004550.5
(22) Anmeldetag: 17.06.2012
(51) Int. Cl.: B01D 53/00, A61L 9/20, B01J 19/12, A62D 3/176

(54) **UV-Strahlermodul für eine Abluft-, Abgas- oder Raumluftreinigungsvorrichtung**

(71) Anmelder: UV-Consulting Peschl e. K., 55130 Mainz (DE)
(72) Erfinder: Peschl, Alexander, 55130 Mainz (DE); Braun, André, 31620 Cépet (FR); Peschl, Günther, 55130 Mainz (DE)
(74) Vertreter: Straub, Bernd

(57) **Zusammenfassung**

Die vorliegende Erfindung betrifft ein Strahlermodul, das zur Verwendung in einer Abluft-, Abgas- oder Raumluftreinigungsvorrichtung auch bei explosionsfähigen Gasgemischen geeignet ist. Das Strahlermodul umfasst zumindest eine Strahlereinheit (1), die eine koaxial in einem Quarzglas-Hüllrohr (3) angeordnete Strahlungsquelle (2) aufweist. Die Strahlereinheit (1) weist ferner ein Kopfteil (10) auf, das mit dem Hüllrohr (3) abdichtend und federnd gelagert verbunden ist und durch das sich eine Schutzgaseinleitungsvorrichtung (6') in einen Raum zwischen dem Hüllrohr (3) und der Strahlungsquelle (2) erstreckt. Das Strahlermodul weist zudem eine Schutzgasüberdruckeinrichtung auf, die über die Schutzgaseinleitungsvorrichtung (6') in dem Raum zwischen dem Hüllrohr (3) und der Strahlungsquelle (2) eine Schutzgasatmosphäre bereitstellt, die einen Überdruck zum Umgebungsdruck von zumindest 0,8 mbar aufweist. Ferner wird eine entsprechende Abluft-, Abgas- oder Raumluftreinigungsvorrichtung offenbart.

## Beschreibung

Die Erfindung betrifft ein UV-Strahlermodul und eine Abluft-, Abgas- oder Raumluftreinigungsvorrichtung, die ein solches UV-Strahlermodul aufweist.

Die Reinigung von Abluft und Abgasen wird vom Gesetzgeber vorgeschrieben, um schädliche Auswirkungen auf die Umwelt zu verringern bzw. vermeiden. Auch ist in vielen Gebieten die Reinigung von Raumluft wünschenswert, wie etwa die Entfernung von Keimen aus hygienischen Gründen oder die Erzeugung von Reinluft, die z. B. bei verschiedenen Produktionsprozessen erforderlich sind.

Zur Abluft-, Abgas- oder auch Raumluftreinigung werden herkömmlicher Weise z. B. Filter und Wäscher sowie diverse technischen Anlagen und Methoden zur thermischen und katalytischen Nachverbrennung eingesetzt. Ferner werden photokatalytische Verfahren zur Luftreinigung verwendet:

Aus DE 20 011 151 U1 ist eine Vorrichtung zur Reinigung von Abluft bekannt, die eine Einrichtung zur Bestrahlung der Abluft mit UV-Licht und eine der UV-Bestrahlungseinrichtung in Strömungsrichtung der Abluft nachgeordnete Filter-/ Katalysatoreinrichtung umfasst, wobei die UV-Bestrahlungseinrichtung und die Filter-/Katalysatoreinrichtung in einem Lüftungskanalabschnitt aufgenommen sind, indem zwischen einem Lufteinlass und einem Luftauslass eine Umlaufströmung der zu reinigenden Abluft gebildet wird.

Weiter ist die oxidative TiO₂-Photokatalyse schon seit über 30 Jahren Forschungsthema und hat in der Applikation zur Behandlung von Abwasser und Abluft Eingang gefunden.

Dabei werden die auf dem Photokatalysator adsorbierten organische Moleküle in Gegenwart von Luftsauerstoff oxidiert, doch ist eine Mineralisierung, d. h. eine vollständig bis zu Kohlendioxid (CO₂), Wasser (H₂O) und ggf. weiteren mineralischen, ungiftigen Produkten verlaufende Oxidation von der Adsorption der intermediär gebildeten Oxidationsprodukte abhängig. Somit werden die Schadstoffe oxidiert bzw. oxidativ abgebaut und nicht in eine andere Phase verlagert, wie dies z. B. bei Absorptionsverfahren oder beim Strippen der Fall ist.

Verschiedene Firmen bieten auch TiO₂-funktionalisierte Trägermaterialien an, die in Modulen zur Ab- und Raumluftbehandlung verwendet werden. Dabei handelt es sich um mit TiO₂ beschichtete Platten, Fasern oder Wabenstrukturen, die in einem von der zu reinigenden Luft durchströmten Behälter angeordnet und von einer Lichtquelle bestrahlt werden.

Ein Problem bei der oxidativen TiO₂-Photokatalyse ist das unterschiedliche Adsorptionsverhalten von Ausgangsverbindungen (Substrat) und intermediären Oxidationsprodukten, und damit die unterschiedliche Geschwindigkeit der verschiedenen radikalisch initiierten Reaktionen, über die im allgemeinen eine vollständige Oxidation (Mineralisierung) erfolgt. Ein unvollständiger Schadstoffabbau mit z. T. toxischen Zwischenprodukten kann bei ungenügender Adsorption und kurzen Verweilzeiten die Toxizität des zu reinigenden Luftstroms vergrößern. Um dies zu vermeiden, wird der Luftstrom bisweilen im Kreislauf geführt, bis der gewünschte Reinigungsgrad erzielt ist.

So weist die photokatalytische Luftreinigung bislang nachteilig eine geringe Effizienz auf, die durch eine geringe Reaktionsfläche im Reaktionsraum, mit kleinen Verweilzeiten im Reaktionsraum und mit der unvollständigen Oxidation und mit dem Austragen von Primärprodukten mit dem Luftstrom mit bedingt ist.

Um diesen Nachteilen zu begegnen, offenbart die DE 10 2005 028 660 A1 ein Verfahren und ein Reinigungssystem, bei dem die zu reinigenden gasförmigen und/oder wässrigen Phasen in einem Reaktionsraum mit einer photokatalytisch wirksamen Titandioxid-Oberfläche in Kontakt gebracht werden, wobei die Titandioxid-Oberfläche aus einem System von Platten und/oder Füllkörpern besteht, die in beliebigen geometrischen Formen vorliegen und konstruktiv so angeordnet sind, dass sie ein hohes Oberflächen/Volumenverhältnis gewährleisten. Die photokatalytisch wirksame Oberfläche wird kontinuierlich mit einer Spülflüssigkeit oder mit der zu reinigenden wässrigen Phase besprüht und die photochemisch katalysierte Radikal-Reaktion mit einer Lichtquelle initiiert. Gegebenenfalls wird in einer Gasphase erzeugtes Ozon zur verstärkenden Radikalbildung zugeführt.

Aktuell werden in der photochemischen oxidativen Abluftreinigung als Strahlungsquellen hauptsächlich Hg-Niederdrucklampen verwendet, bei denen die Emission bei 254 nm zur elektronischen Anregung des Photokatalysators genutzt wird. Im Allgemeinen können für die TiO₂-Photokatalyse Strahlungsquellen eingesetzt werden, die im vom UV-C (200 nm) bis UV-A- (380 nm) Spektralbereich emittieren.

Neben der elektronischen Anregung des Photokatalysators und der Photolyse der Schadstoffe bei 254 nm kann Strahlung im VUV-Spektralbereich, z. B. bei 185 nm für Hg-Niederdrucklampen, durch den in der Luft enthaltenen Sauerstoff absorbiert werden, und so Ozon erzeugt werden, das den oxidativen Schadstoffabbau unterstützt.

Grundsätzlich jedoch ist der Einsatz photochemischer, photo-initierter und photokatalytischer Prozesse bei der Gasreinigung bislang nicht möglich, wenn der Gasstrom ein explosionsfähiges Gemisch enthält, da einerseits an der Oberfläche der UV-Strahler Temperaturen herrschen können, die eine Selbstzündung des Gemischs hervorrufen können, sowie bei einem eventuell auftretenden Versagen bzw. Bruch der Strahlungsquelle Funken das Gemisch entzünden können.

Ausgehend von diesem Stand der Technik liegt der vorliegenden Erfindung die Aufgabe zugrunde, eine UV-Strahlungsvorrichtung zu schaffen, die den ATEX-Richtlinien genügt und damit in photochemischen, photo-initiierten, photokatalytischen oder kombinierten Abluftreinigungsprozessen eingesetzt werden kann.

Diese Aufgabe wird durch ein Strahlermodul mit den Merkmalen des unabhängigen Anspruchs 1 gelöst.

Eine weitere Aufgabe liegt in der Schaffung einer Abluft-, Abgas- oder Raumluftreinigungsvorrichtung, mit der auch explosionsfähige Stoffströme photochemisch, respektive photooxidativ, etwa durch Photolyse, und/oder photooxidativ behandelt werden können, und die ferner eine hohe Effizienz mit einfacher Konstruktion und Wartung verbindet.

Diese Aufgabe wird durch eine Abluft-, Abgas- oder Raumluftreinigungsvorrichtung mit den Merkmalen des Anspruchs 7 gelöst.

Bevorzugte Ausführungsformen der Vorrichtungen werden durch die Unteransprüche beschrieben.

Ein erfindungsgemäß ausgeführtes Strahlermodul, das zur Verwendung in einer Abluft-, Abgas- oder Raumluftreinigungsvorrichtung geeignet ist, besteht aus einer oder mehreren Strahlereinheiten. Jede Strahlereinheit weist eine koaxial in einem Hüllrohr aus Quarzglas angeordnete Strahlungsquelle auf. Die Strahlereinheit weist ferner ein Kopfteil auf, das mit dem Hüllrohr abdichtend und federnd gelagert verbunden ist. Eine Schutzgaseinleitungsvorrichtung erstreckt sich durch das Kopfteil in den Raum zwischen Hüllrohr und Strahlungsquelle. Schließlich weist das Strahlermodul eine Schutzgasüberdruckeinrichtung auf, die über die Schutzgaseinleitungsvorrichtung in den Raum zwischen dem Hüllrohr und der Strahlungsquelle eine Schutzgasatmosphäre bereitstellt, die einen Überdruck zum Umgebungsdruck von zumindest 0,8 mbar aufweist, so dass kein atmosphärischer Sauerstoff in diesen Raum eindringen kann und auch bei einem Defekt des Hüllrohrs eine Schutzatmosphäre um die Strahlungsquelle aufrechterhalten wird, die eine Bildung explosionsfähiger Gasgemische verhindert.

Das Hüllrohr wird bevorzugt aus geeignetem und auf dem Markt erhältlichem synthetischen Quarz gefertigt sein. Bevorzugt wird aus Gründen der mechanischen Stabilität vorgeschlagen, eine besonders dicke Wandstärke von zumindest 3 mm zu wählen und ein vorzugsweise mehrmals getempertes Quarz einzusetzen, um eine größtmögliche Spannungsfreiheit im Hüllrohr zu erzielen. Ferner soll das Hüllrohr einer Druckprüfung mit 10 bar standhalten.

Um sicherzustellen, dass die Strahlungsquelle in dem Hüllrohr komplett von Schutzgas umspült wird, kann sich von der Schutzgaseinleitungsvorrichtung ein Kapillarrohr zwischen dem Hüllrohr und der Strahlungsquelle bis zum entgegengesetzt liegenden Ende des Hüllrohrs erstrecken und dort einen Auslass für das Schutzgas in den Raum zwischen dem Hüllrohr und der Strahlungsquelle bereitstellen. Ferner ist in dem Kopfteil eine Schutzgasausleitungsvorrichtung vorgesehen. Die Schutzgasüberdruckeinrichtung weist eine mit der Schutzgaseinleitungsvorrichtung verbundene Schutzgaszuleitung und eine mit der Schutzgasausleitungsvorrichtung verbundene Schutzgasableitung auf, in der eine Druckmessvorrichtung vorgesehen ist, die mit einem Steuergerät verbunden ist, das ein in der Schutzgaszuleitung angeordnetes Magnetventil, das bevorzugt ein Proportionalventil ist, steuert.

Falls das Strahlermodul eine mehrere Strahlereinheiten umfasst, kann die Schutzgasleitung von der Schutzgaszuleitung parallel oder bevorzugt in Reihe über die Schutzgaseinleitungsvorrichtung und Schutzgasausleitungsvorrichtung der Strahlereinheiten zu der Schutzgasableitung geführt sein.

Das den Schutzgasdruck regelnde Steuergerät ist in einer bevorzugten Ausführungsform mit einem die Strahlungsquelle mit Strom versorgendem Vorschaltgerät verbunden, um die Strahlungsquelle abzuschalten, wenn durch die Druckmessvorrichtung ein Druck unterhalb des Mindestüberdrucks von 0,8 mbar und oberhalb eines vorab festgelegten zulässigen Maximalüberdrucks festgestellt wird.

Das Kopfteil der Strahlereinheit kann mittels eines Ringflansches mit dem Hüllrohr verbunden sein, wobei der Ringflansch bevorzugt mit einer konischen Innenfläche ausgebildet ist, die mit dem konisch ausgebildeten offenen Ende des Hüllrohrs korrespondiert. Der Konus verjüngt sich zu der von dem Kopfteil weg weisenden Seite.

Zur Abdichtung der Verbindung Hüllrohr-Ringflansch können an der Innenfläche des Ringflansches zwei oder gegebenenfalls auch mehr umlaufende Nuten zur Aufnahme von Dichtringen vorgesehen sein.

Ferner kann der Ringflansch zur Befestigung der Strahlereinheit in oder an einer Abluft-, Abgas- oder Raumluftreinigungsvorrichtung ausgebildet sein und hierzu z. B. Durchtrittsöffnungen für Schrauben aufweisen.

Das Kopfteil kann becherartig ausgebildet sein und an seinem offenen Ende einen nach außen weisenden Kragen aufweisen, mit dem das Kopfteil an dem Hüllrohr anliegt. Um auch hier einen dichten Übergang zu schaffen, kann die durch den Kragen gebildete Stirnfläche des Kopfteils eine Ringnut aufweisen, in die ein Dichtring aufgenommen wird.

Um die zentrierte Anordnung des Kopfteils auf dem in dem Ringflansch gehaltenen Hüllrohr zu erleichtern, kann der Ringflansch einen zum Kopfteil weisenden ringförmigen Überstand aufweisen, der zumindest teilweise in eine weitere auf der Stirnfläche des Kragens angeordnete Ringnut hineinragt. Dieser ringförmige Überstand kann ferner dem andauernden strahlungsdichten Abschluss nach außen hin dienen.

Die federgelagerte Verbindung des Kopfteils mit dem Hüllrohr wird dabei über eine an bzw. auf dem Kragen anliegenden Scheibe bewerkstelligt, die über Federschrauben mit dem Ringflansch verbunden ist. Die federgelagerte Verbindung ist vorteilhaft, um die etwa bei Temperaturschwankungen auftretenden unterschiedlichen Ausdehnungen (insbesondere bei Montage des Kanals im Außenbereich) aufzufangen, damit sich die Schraubverbindungen nicht lösen können.

Eine erfindungsgemäße Abluft-, Abgas- oder Raumluftreinigungsvorrichtung, die in einem Abgas- oder Lüftungskanalabschnitt einer Abgas-/Lüftungsanlage mit einem Einlass und einem Auslass für eine zu reinigende Abluft-, Abgas- oder Raumluft untergebracht ist, weist ein erfindungsgemäßes Strahlermodul zur photochemischen, photoinitiierten und/oder photokatalytischen oxidativen Behandlung des zu reinigenden Abluft-, Abgas- oder Raumluftstroms auf, der nun auch Substanzen enthalten kann, die explosionsfähige Gasgemische bilden können.

In Weiterbildungen kann die Abluft-, Abgas- oder Raumluftreinigungsvorrichtung stromaufwärts des Strahlermoduls einen Filter aufweisen sowie alternativ oder zusätzlich stromabwärts des Strahlermoduls einen Aktivkohlefilter. Ferner kann ein zusätzlicher Ozonierungsraum vorgesehen sein, der stromabwärts des Strahlermoduls und stromaufwärts des Aktivkohlefilters angeordnet wird.

Ist ein Ozonierungsraum vorgesehen, weist die Vorrichtung eine Ozon-Zufuhrvorrichtung auf, die mit einer vorzugsweise außerhalb des Lüftungskanalabschnitts vorliegenden Ozonquelle verbunden ist und die bevorzugt ein perforiertes, senkrecht zur Strömungsrichtung in dem Ozonierungsraum angeordnetes ringförmiges Glasrohr aufweist. Dabei kann die Größe des Ozonierungsraums an eine Schadstoffbeladung des Abluft-, Abgas- oder Raumluftstroms durch Variation des Abstands zwischen dem Strahlermodul und dem Aktivkohlefilter angepasst werden.

Weist die Vorrichtung einen Ozonierungsraum auf, oder werden im Strahlermodul Ozon erzeugende Strahlungsquellen verwendet, kann die Vorrichtung stromabwärts dazu und bevorzugt stromabwärts des Aktivkohlefilters einen Ozonsensor aufweisen, der zum Abschalten der Strahlungsquelle und/oder der Ozon-Zufuhrvorrichtung bei einer durch den Ozonsensor festgestellten Ozonkonzentration oberhalb eines vorgegebenen Grenzwerts mit dem Vorschaltgerät der Strahlungsquelle und/oder der Ozon-Zufuhrvorrichtung gekoppelt ist.

Neben einem Ozonsensor kann die Vorrichtung ausgangseitig alternativ oder zusätzlich mit einem Temperatursensor ausgestattet sein, der zum Abschalten der Strahlungsquelle bei einer durch den Temperatursensor festgestellten Temperatur des Abluft-, Abgas- oder Raumluftstroms oberhalb eines vorgegebenen Grenzwerts mit dem Vorschaltgerät der Strahlungsquelle gekoppelt ist.

Schließlich können in einer Ausführungsform, in der das Strahlermodul zur photokatalytischen oder kombiniert photooxidativen und photokatalytischen Behandlung des Gasstroms eingesetzt werden soll, die Strahlereinheiten des Strahlermoduls in einer oder in mehreren Ebenen senkrecht zur Strömungsrichtung des Abluft-, Abgas- oder Raumluftstroms angeordnet sein, wobei beidseitig zu jeder Strahlerebene senkrecht zur Strömungsrichtung Photokatalysatorschichten angeordnet sind, die Trägerkörper mit einer Titandioxid-Oberfläche aufweisen, wobei die Trägerkörper Quarz- oder Glasfasern oder Glas- oder Silikagelpartikel oder Keramikpartikel sind, die mit Titandioxid beschichtet sind.

Dabei kann jede Photokatalysatorschicht durch ein Netzstrukturenpaar mit dem Innenquerschnitt des Lüftungskanalabschnitts entsprechenden Abmessungen begrenzt werden, zwischen denen die Trägerkörper gepackt sind. Die Packung der Trägerkörper hat einen Druckverlust von maximal 500 Pa.

Weitere Ausführungsformen, sowie einige der Vorteile, die mit diesen und weiteren Ausführungsformen verbunden sind, werden durch die nachfolgende ausführliche Beschreibung deutlich und besser verständlich. Unterstützend hierbei ist auch der Bezug auf die Figuren in der Beschreibung. Gegenstände oder Teile derselben, die im Wesentlichen gleich oder sehr ähnlich sind, können mit denselben Bezugszeichen versehen sein.
**Fig. 1** zeigt eine Seitenschnittansicht eines UV-Strahlermoduls nach einer erfindungsgemäßen Ausführungsform.
**Fig. 2** zeigt eine weitere Seitenschnittansicht des UV-Strahlermoduls.
**Fig. 3** zeigt eine Seitenschnittansicht durch ein Strahlermodul einer Abluft-, Abgas- und Raumluftreinigungsvorrichtung mit erfindungsgemäßen UV-Strahlermodulen.
**Fig. 4** zeigt eine perspektivische Ansicht des Strahlermoduls mit einer von einem Deckel verschlossenen Revisionsöffnung im Lüftungskanalabschnitt.

Die Erfindung betrifft ein Strahlungsmodul, das einen oder mehrere explosionsgeschützte UV-Strahler für Gasphasenreaktoren aufweist. Der Zündschutz wird durch eine Überdruckkapselung mit inertem Schutzgas, vorzugsweise Stickstoff erzielt.

Als UV-Strahlungsquellen können Quecksilberdampfniederdruck-Strahlenquellen, mit und ohne Amalgam, zum Einsatz kommen, mittels derer in der Gasphase durch Photolyse, photoinitiiert und/oder photokatalytisch und/oder oxidativ (in Verbindung mit Titandioxid-Katalysatoren in Filterform) unerwünschte Gerüche, chemische Verbindungen (VOCs) sowie Fettaerosole abgebaut werden können.

Bei der photochemischen oxidativen Abluftbehandlung werden gasförmige Moleküle, partikuläre Aerosole und Moleküle, die in Aerosoltröpfchen enthalten sind durch UV-Strahlung photolysiert, und am TiO₂-Photokatalysator adsorbierte Moleküle oxidiert. Bei Strahlungen mir einer Wellenlänge unter 200 nm (konkret bei ozonbildenden Niederdruckstrahlenquellen 185 nm) führt die Photolyse zur Fragmentierung dieser Moleküle und/oder zur Bildung von OH-Radikalen aus wässrigen Aerosoltröpfchen. Bei Strahlung im VUV-Bereich entsteht weiterhin Ozon, das ebenfalls mit den im Luftstrom enthaltenen organischen Molekülen reagieren kann. Die durch HO-Radikale und Ozon eingeleiteten Oxidationsreaktionen bezeichnet man in diesem Fall als photochemisch initiierte Reaktionen. Strahlung vom VUV- bis zum UV-A-Spektralbereich wird auch zur elektronischen Anregung des TiO2-Photokatalysators verwendet, die zur photokatalysierten Oxidation der adsorbierten Schadstoffmoleküle führt.

Bei der photokatalytischen Abluftbehandlung werden UV-Strahlenquellen eingesetzt, welche in der spektralen Absorptionskurve von Titandioxid als Katalysator emittieren. Hierfür können ozonfreie UV-Strahlenquellen mit emittierter UV-Strahlung > 200nm eingesetzt werden.

Zur kombinierten photochemischen, photo-initiierten und photokatalytischen oxidativen Abluftbehandlung werden UV-Strahlenquellen eingesetzt, die sowohl unter 200 nm als auch oberhalb 200 nm emittieren, z. B. Hg-Niederdruckdampflampen mit oder ohne Amalgam mit den Hauptemissionslinien bei 185 nm und 254 nm.

Der oder die UV-Strahler des erfindungsgemäßen Strahlermoduls sind so ausgeführt, dass sie den ATEX-Richtlinien genügen und so zur Abluftreinigung auch in zündfähiger Atmosphäre eingesetzt werden. Ferner umfasst das Strahlermodul eine entsprechende Steuerung zum sicheren Betrieb. Das Strahlermodul ist bei entsprechender Auswahl der Strahlungsquellen für rein photochemische, photoinitiierte und/oder photokatalytische bzw. kombinierte Prozesse geeignet.

Anders als bei den UV-Tauchlampen für die Flüssigphase können für die Abluftaufbereitung in der Gasphase keine Hg-Mitteldruckstrahlenquellen eingesetzt werden, da diese im Betrieb eine Oberflächentemperatur von ca. 800 °C - 950 °C aufweisen. Hingegen liegt die Oberflächentemperatur der Niederdruck-Strahlenquellen deutlich niedriger bei ca. 40 °C (ohne Amalgam) bzw. 90 °C - 100 °C mit Amalgam.

Die Ausführung der UV-Strahler, um diese in einer ATEX-Zone betreiben zu können, muss einen sicheren Betrieb gewährleisten.

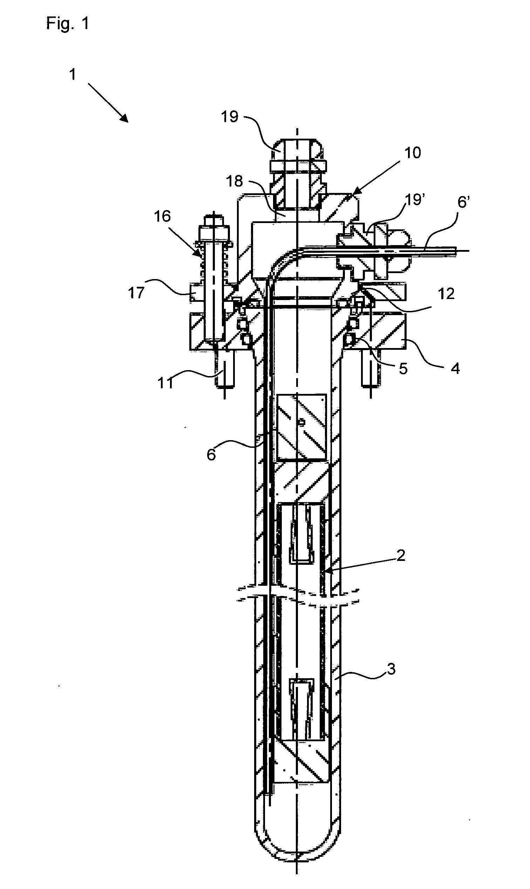

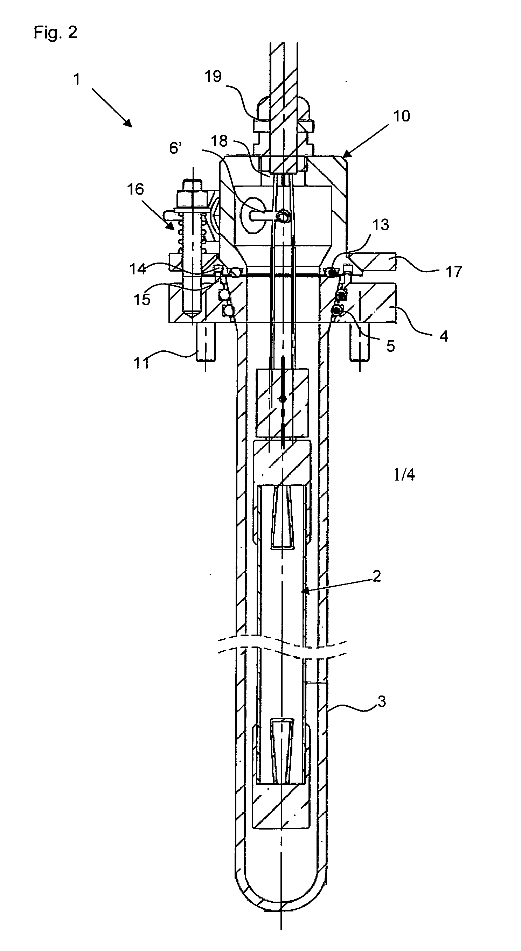

Eine erfindungsgemäße Ausführungsform der UV-Strahlereinheit 1 eines Strahlermoduls ist in **Fig. 1** **und** **2** dargestellt. Um zu vermeiden, dass der Gasstrom in direkten Kontakt mit der Strahleroberfläche kommt und um den Strahler 2 vor etwaiger Verschmutzung und Beschädigung zu schützen, wird der Strahler 2 in einem Hüllrohr 3 aus natürlichem Quarzglas bei Niederdruckstrahlenquellen aus dotiertem Quarz oder aus synthetischem Quarz bei Niederdruckstrahlenquellen aus natürlichem bzw. synthetischem Quarzglas angeordnet, das in dem eingesetzten Wellenlängenbereich transparent ist. Das Quarzrohr 3 weist eine hohe Wandstärke von zumindest 3 mm auf, um eine erhöhte mechanische Stabilität zu erreichen. Zur Erzielung einer größtmöglichen Spannungsfreiheit wird das Quarzrohr mehrfach getempert.

Die Halterung des Hüllrohres 3 am Kopfteil 10 ist vorliegend in einem Flanschkonzept realisiert, welches keine großen Spannungen durch seitliche Belastungen erzeugen kann und nur einen kleinen Winkel am Konusflansch aufweist.

Das in **Fig. 1** **und** **2** dargestellte Flanschkonzept umfasst einen Flanschring 4 mit einer konischen Innenfläche, in dem das korrespondierend dazu konisch ausgebildete offene Ende des Hüllrohrs 3 aufgenommen wird. Hierbei wird bevorzugt ein "negativer" Konusflansch 4 eingesetzt, bei dem sich der Konus auf von dem Kopfteil 10 weg weisenden Seite verjüngt und sich der Außenumfang des Hüllrohrendes zu dessen Öffnung hin aufweitet.

Selbstverständlich können auch Flanschringe mit zylindrischer Aufnahmeöffnung wie bei Bundflanschen der Fall eingesetzt werden, bevorzugt sind aber Flanschkonzepte, die wie der Konusflansch 4 nicht nur eine kraftschlüssige, sondern auch eine formschlüssige Verbindung bereitstellen, damit das Hüllrohr 3 unter keinen Umständen, etwa durch Vibrationen, aus dem Kopfteil in den Reaktionsraum rutschen kann, da nicht immer gewährleistet sein kann, dass das Hüllrohr 3 beidseitig gehaltert wird.

Die Abdichtung zwischen Flansch 4 und Hüllrohr 3 wird über zwei Dichtungsebenen mittels Dichtringen 5, die in umlaufende Nuten in der Konusfläche angeordnet sind, bereitgestellt, die gleichzeitig die seitliche Druckübertragung auffangen, ohne das Hüllrohr 3 unzulässig zu überlasten.

Die verwendeten Quarzhüllrohre 3 sollten mit mindestens 10 bar druckgeprüft sein.

Eine weitere erfindungsgemäße Maßnahme zur explosionsgeschützen Gestaltung des Strahlermoduls, so dass dessen Einsatz in der Abluftreinigung auch bei explosionsfähigen Gemischen ermöglicht wird, liegt in der Realisierung einer Schutzgasspülung. Dazu weist das Strahlermodul eine Schutzgasüberdruckeinrichtung auf, die über die Schutzgaseinleitungsvorrichtung in dem Raum zwischen dem Hüllrohr und der Strahlungsquelle eine Schutzgasatmosphäre bereitstellt, die einen Überdruck zum Umgebungsdruck von zumindest 0,8 mbar aufweist, so dass kein atmosphärischer Sauerstoff in diesen Raum dringen kann und auch bei einem Defekt des Hüllrohrs eine Schutzgasatmosphäre um die Strahlungsquelle aufrecht erhalten bleibt, die eine Bildung explosionsfähiger Gasgemische verhindert.

Das Schutzgas (vorzugsweise N₂) wird über eine Schutzgaseinleitungsvorrichtung 6' durch das Kopfteil 10 und über ein Kapillarrohr 6, das z. B. aus Edelstahl bestehen kann, zwischen Strahler 2 und Hüllrohr 3 nach unten zum geschlossenen Ende des Hüllrohrs 3 geführt, um eine vollständige Spülung des Zwischenraums zwischen Strahler 2 und Hüllrohr 3 zu gewährleisten. Das Kapillarrohr 6 ist im Kopfteil 10 befestigt, vorzugsweise formschlüssig, um dessen Herausrutschen zu vermeiden.

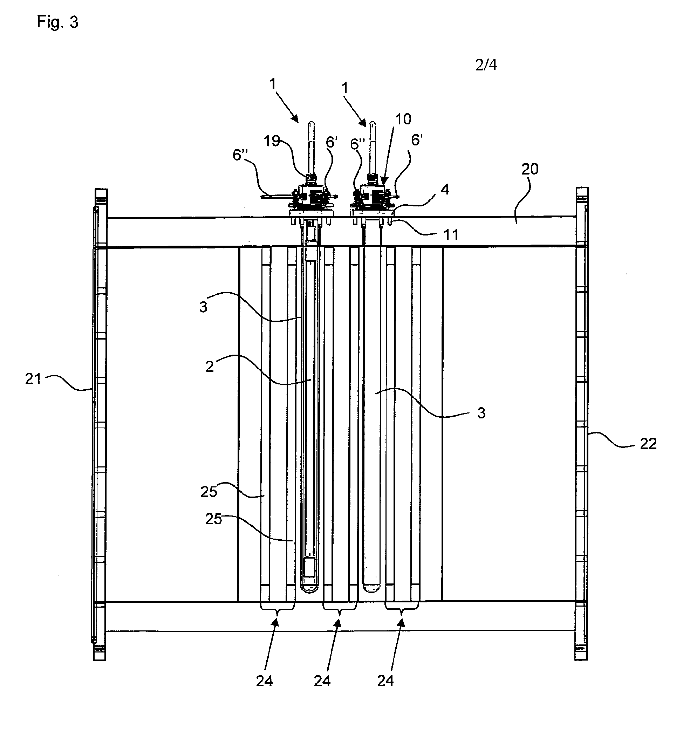

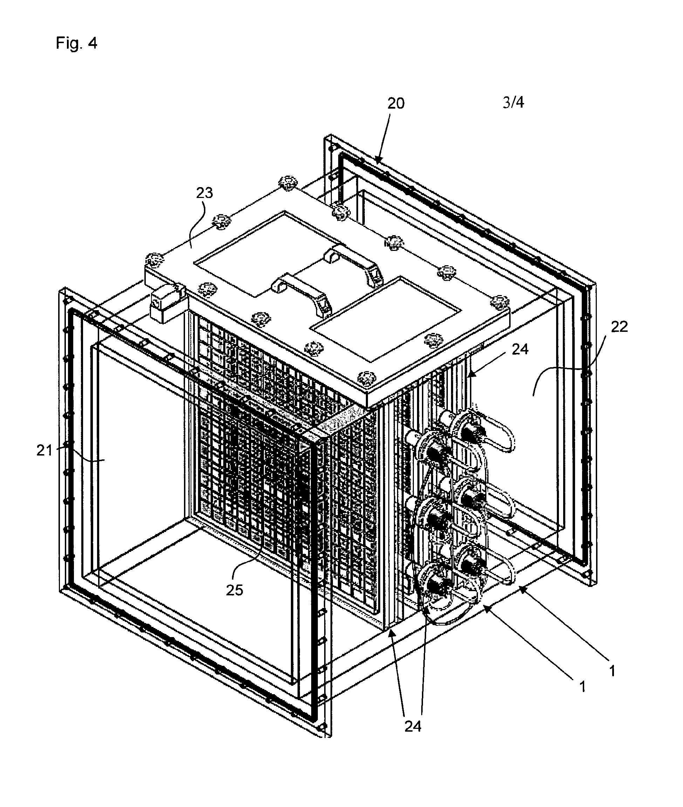

Weist das in einem Abluftreinigungsmodul verwendete Strahlermodul mehr als eine Strahlereinheit 1 auf, wie. z. B. in **Fig. 4** gezeigt, sechs Strahlereinheiten 1 in zwei Ebenen, kann das Strahlermodul ein Schutzgasleitungssystem beinhalten, mit dem die Strahlereinheiten 1 parallel oder bevorzugt in Reihe durchspült werden. Das in Reihe geschaltete Schutzgasleitungssystem führt von der Schutzgaseinleitungsvorrichtung 6' einer ersten Strahlereinheit 1 über dessen Schutzgasausleitungsvorrichtung 6" (zu sehen in **Fig. 3****)** zur Schutzgaseinleitungsvorrichtung 6' einer zweiten Strahlereinheit 1 und so weiter bis zur Schutzgasausleitungsvorrichtung 6" der letzten Strahlereinheit 1. Gegebenenfalls und eventuell in Abhängigkeit der Anzahl der verwendeten Strahlereinheiten 1 im Strahlermodul kann die Schutzgasleitung kombiniert parallel und in Reihe erfolgen, z. B. wenn das Strahlermodul mehrere Strahlerebenen aufweist, die wiederum mehrere Strahlereinheiten 1 umfassen.

Werden mehrere Strahlereinheiten 1 in Reihe durchspült, so befindet sich an der Schutzgasausleitungsvorrichtung an der letzten Strahlereinheit 1 der Reihe ein Drucksensor, der Signale an ein Steuergerät liefert, welches ein Schutzgasventil (z. B. ein Proportionalventil) an der Schutzgaseinleitungsvorrichtung in die erste Strahlereinheit 1 regelt.

Innerhalb des Schutzgassystems wird ein Überdruck zur Umgebung des Moduls von größer/gleich 0,8 mbar eingestellt bzw. geregelt und überwacht. Vorzugsweise wird ein Steuergerät verwendet, welches vor Inbetriebnahme das Schutzgasleitungssystem mit dessen 5-fachen Volumen durchspült und anschließend in eine Betriebsweise der Leckageausgleichskompensation schaltet, bei der Schutzgasverluste durch Nachregelung mittels des eingangsseitigen Proportionalventils ausgeglichen werden und ein Betriebsüberdruck im vorliegenden Fall im Bereich von 2 bis 5 mbar und während der Vorspülphase im Bereich von 6 bis 14 mbar aufrecht erhalten wird.

Das Steuergerät ist mit einer Schutzschaltung ausgestattet, die verhindert, dass die Strahlenquelle bei zu hohem Überdruck beschädigt oder zerstört wird. Dazu kann ein maximal zulässiger Überdruck eingestellt werden, der beispielsweise bei 15 mbar liegen kann.

Alternativ kann auch kontinuierlich gespült werden, wobei allerdings die Strahlungsquellen unter Umständen derart gekühlt werden, dass sie zu kalt betrieben werden. Dies kann jedoch gegebenenfalls zweckmäßig sein, wenn etwa eine thermische Anpassung der Lampe erfolgen soll. Die thermische Anpassung wird mit der kontinuierlichen Schutzgasspülung je nach Auslegung entsprechend ausgewählt.

Wird ohne Steuergerät gearbeitet, ist es erforderlich, die Schutzgasspülung durch Messung des Durchflusses im Einlass sowie im Auslass in Verbindung mit einer Druckmessung zu überwachen.

Durch die Art der Schutzgasspülung und Überwachung ist gewährleistet, dass es bei einer Blockierung oder einem Bruch der Schutzgasleitungen zu einer Zwangsabschaltung der Strahler kommt, so dass ein Betrieb der Strahler ohne Schutzgasatmosphäre verhindert wird.

Gegebenenfalls können im Schutzgasspülsystem auch mehrere Sensoren, z. B. an verschiedenen Stellen eingesetzt werden um eine zuverlässige Spülung sicherzustellen.

Das Kopfteil 10 dient, wie in **Fig. 3** zu sehen, der Befestigung der Strahlereinheiten 1 in einem Rahmen oder Kanalabschnitt 20, der in einer Lüftungsanlage vorgesehen oder nachgerüstet wird. Dazu können Schrauben 11 eingesetzt werden, die durch den Flanschring 4 in den Rahmen oder Kanalabschnitt 20 eingreifen.

Das Kopfteil 10 kann aus Edelstahl gefertigt sein und ist so ausgeführt, dass keine UV-Strahlung nach außen tritt. Das Kopfteil 10 ist in **Fig. 1** und **2** becherartig ausgeführt und dichtet das Hüllrohr 3 gegenüber der Umgebung ab. Das Kopfteil 10 weist an seinem offenen, dem Hüllrohr 3 zugewandten Ende einen nach außen weisenden Kragen 12 auf. Die Öffnung des Kopfteils 10 fluchtet mit dem Innendurchmesser des Hüllrohrs 3, wobei die Stirnfläche des Kopfteils 10, die durch den Kragen 12 gebildet wird, eine Ringnut aufweist, um einen Dichtring 13 zur Abdichtung gegenüber dem Hüllrohr 3 aufzunehmen. Eine weitere, radial weiter außen auf der Stirnfläche des Kragens 12 angeordnete Ringnut 14 ist vorgesehen, zumindest teilweise einen korrespondierend ausgebildeten ringförmigen Überstand 15 des Konusflansches 4 aufnehmen zu können.

Das Kopfteil 10 wird über federgelagerte Schraubverbindungen 16 zwischen einer auf dem Kragen 12 des Kopfteils 10 aufliegenden Scheibe 17 und dem Konusflansch 4 gesichert, damit etwa die bei Temperaturschwankungen auftretenden unterschiedlichen Ausdehnungen (insbesondere bei Montage des Kanals im Außenbereich) aufgefangen werden und die Schraubverbindungen sich nicht lösen können.

Für den elektrischen Anschluss der Strahlungsquelle 2 ist im "Becherboden" des Kopfteils 10, an dem dem Kragen 12 entgegengesetzten Ende, eine Durchtrittsöffnung 18 vorgesehen, in der ein entsprechendes Anschlussstück 19, durch das die elektrischen Leitungen zu der Strahlungsquelle geführt werden, angeordnet ist.

Ferner kann vorgesehen sein, dass das Kopfteil 10 intern über eine Erdungsschraube geerdet wird und in der Schutzart IP68 ausgeführt ist, d. h. dass eine staub- und wasserdichte Abdichtung bereitgestellt wird.

Mit der Einteilung elektrischer Betriebsmittel in Temperaturklassen werden explosionsgeschützte Betriebsmittel in ihren Oberflächentemperaturen so ausgelegt, dass die für die Temperaturklasse typische max. Oberflächentemperatur garantiert wird und damit eine Oberflächentemperaturzündung ausgeschlossen wird. Die Angabe der max. Oberflächentemperatur enthält einen Sicherheitsabstand zur kleinsten Zündtemperatur.

Zur Einhaltung der ATEX-Richtlinien ist die maximale Oberflächentemperatur, insbesondere am Hüllrohr zu definieren (gem. Auslegung der zulässigen Gasstromtemperatur, der daraus resultierenden zulässigen Hüllrohrtemperatur abzgl. der relevanten Sicherheitsabschläge zur Erreichung der Einstufung z.B. der Temperaturklasse T3, < 200 °C). Dazu wird der Maximalstrom am Vorschaltgerät, mit dem der oder die Strahler betrieben werden, vorgegeben und die Vorschaltgerätetechnik ist entsprechend zu begrenzen, damit es im Fehlerfall nicht zur Überhitzung der Strahlenquelle kommen und die Oberflächentemperatur am Hüllrohr unzulässig ansteigen kann. Ferner muss stromabwärts der photooxidativen und/oder photokatalytischen Behandlung des Gasstroms eine Temperaturüberwachung installiert werden, um sprunghafte Temperaturanstiege zu detektieren, die eine Überhitzung des Systems überwachen und verhindern soll. Wird ein solcher Temperaturanstieg detektiert, können die Strahler unmittelbar abgeschaltet werden, damit keine Entzündung oder Explosion stattfinden kann.

Aus Kostengründen kann anstelle einer Sicherheits-SPS eine fest verdrahtete Steuerung mit Sicherheitsrelais verwendet werden. Sicherheitsrelevante Überwachungsfunktionen wie z. B. der Schutzgasüberdruck und die Ablufttemperatur werden zumindest zweikanalig ausgeführt, so dass sich die Systeme gegenseitig prüfen und eventuelle Fehler erkennen können, wohingegen einkanalige Systeme auf Fehler in der Regel mit einem Versagen reagieren.

Bei einem Strahlermodul, das mehrere Strahlereinheiten umfasst, kann es zweckmäßig sein, deren Steuerung in Gruppen auszubauen, damit je nach Beladung der Gasphase bzw. Abluft die Anzahl der Strahlereinheiten angepasst werden kann, die erforderlich ist, um die enthaltenen organischen Verbindungen abzubauen. So können mit einer derartigen Steuerung Strahlereinheiten zu- oder abgeschaltet werden, wenn die Beladung des Abluftstroms oder auch die zu behandelnde Abluftmenge variiert. Bei geringer Beladung kann die Abluftreinigungsanlage dann energiesparend mit einem Minimum an Strahlern betrieben werden.

Hierbei kann vorgesehen sein, dass die Schutzgaszufuhr entsprechend nur zu den in Betrieb befindlichen Strahlereinheiten erfolgt. Dazu kann es vorteilhaft sein, ein Strahlermodul zu verwenden, das aus mehreren Strahlerebenen mit Strahlereinheiten besteht, wobei jede Strahlerebene ihr eigenes Schutzgassystem aufweist, so dass bei einem ebenenweise Betreiben der Strahlereinheiten jeweils nur das dazugehörige Schutzgassystem aktiv ist.

Beim Auftreten von Fehlermeldungen jedoch werden immer alle Strahlenquellen abgeschaltet.

Die im erfindungsgemäßen Strahlermodul umgesetzten Maßnahmen zum Explosionsschutz umfassen:
1. Die Temperaturklassifizierung durch überprüfte Oberflächentemperaturen sowie korrekte Stromeinstellung am elektronischen Vorschaltgerät, wobei eine maximal zulässige Stromstärke eingestellt wird, die den Betrieb des Strahlermoduls innerhalb der zugelassenen Oberflächentemperaturen sicherstellt. Das Vorschaltgerät wird entsprechend so ausgeführt, dass der Maximalstrom der Strahlenquelle nach dem Zünden bauartbedingt nicht über dem zur Einhaltung der Oberflächentemperatur festgelegten Maximalstrom auf der Sekundärseite betrieben werden kann.
2. Die Schutzgasüberdruckkapselung der Strahlungsquellen beispielsweise mittels eines F850S-Systems von Gönnheimer electronic GmbH, Neustadt, besteht aus mindestens einem Steuergerät und einem Magnetventil, z. B. einem Proportionalventil. Bei der Verwendung von mehreren Strahlereinheiten können diese in Reihe geschaltet werden und mit Schläuchen verbunden werden. Allerdings sollten nicht mehr als sechs Strahlereinheiten mit einem Schutzgassystem in Reihe betrieben werden.
3. Die kontinuierliche Messung der Ablufttemperatur bauseits und Auswertung über die zweikanalig, redundant und mit Sicherheitsrelais ausgeführte Steuerung der Strahlereinheiten.
4. Ausführung des Steuerschrankes fest verdrahtet. Es wird bewusst auf die Verwendung einer SPS verzichtet.
5. Messung der maximal zulässigen Ozonkonzentration im Abluftstrom, sofern erforderlich, d. h. wenn Ozon zur Unterstützung des oxidativen Abbaus zugeführt oder durch eine Strahlungsquelle mit Strahlung > 200 nm erzeugt wird.
6. Ausführung des Hüllrohres aus synthetischem, vorzugsweise mehrfach getempertem Quarz mit besonders dicker Wandstärke.
7. Abschirmung der Außenumgebung mittels stabilen Kopfteils.
8. Abgastemperaturmessung.

Um Betriebspersonal zu schützen, kann das Strahlermodul bzw. die Abluftreinigungsanlage mit Warn- und Sicherheitshinweisen gekennzeichnet sein. Weiter kann in dem entsprechenden Raum oder Gebäude, in dem die Anlage untergebracht ist, zum Schutz vor austretender UV-Strahlung und - sofern oxidativer Betrieb vorgesehen ist - Restozon ein zweikanalig ausgeführter Türkontaktschalter vorgesehen sein, ebenso wie ein Sensor zur Restozonmessung, falls dies in der Anlage verwendet oder erzeugt wird.

Um den sicheren Betrieb des Strahlermoduls in einer Abluftreinigungsanlage sicherzustellen, und dass die UV-Strahlung zur Behandlung in der spezifischen Abluft selbst keine Zündquelle darstellt, kann vorgesehen sein, dass bei jedem Strahlerwechsel, spätestens jedoch nach einer vorgegebenen Zeitspanne z. B. alle 12 Monate, präventiv alle Schutzgasleitungen/Schläuche im Zuge einer Wartung gewechselt werden, um Materialermüdungserscheinungen vorzugreifen.

Der Schaltschrank mit den Steuerungsgeräten wird außerhalb der ATEXklassifizierten Zone angeordnet, wobei eine maximale Leitungslänge zur Strahlereinheit gemäß der Herstellervorgaben der elektronischen Vorschaltgeräte eingehalten wird.

Ferner kann vorgesehen sein, dass das Strahlermodul im Unterdruckbereich eingesetzt wird, damit bei oxidativen Prozessen kein Ozon unkontrolliert in die Umgebung der Abluftanlage abgegeben werden kann.

Zum mechanischen Schutz der Strahlereinheiten wird in den Abluftkanal vor das Strahlermodul ein entsprechender Vorfilter eingesetzt oder anderweitige Maßnahmen bzw. prozessseitige Risikoanalysen vorgenommen, damit keine mechanische Belastung durch Partikel an dem oder den Hüllrohren entstehen können.

Weiterhin ist sicherzustellen, dass in einer Abluft-, Abgas- und Raumluftreinigungsvorrichtung, die das Strahlermodul umfasst, zugeführtes oder entstandenes Ozon den Flammpunkt/Zündpunkt des Gasstroms an einem nachgeschalteten Filter bzw. Katalysator (z. B. TiO₂ auf Trägermaterial) nicht herabsetzt oder es durch ionisierende Strahlung der Strahler an dem Filter/Katalysator zu elektrostatischen Aufladungen kommen kann.

So betrifft eine erfindungsgemäße Vorrichtung eine Abluft-, Abgas- und Raumluftreinigungsvorrichtung, die ein erfindungsgemäßes Strahlermodul umfasst und zur Integration in eine bestehende oder neue Reinigungsanlage geeignet ist und die oben genannten Probleme vermeidet.

Die Reinigungsvorrichtung wird durch einen Lüftungskanalabschnitt 20 mit einem Gas- bzw. Lufteinlass 21 und einem Gas- bzw. Luftauslass 22, dargestellt in **Fig. 3** **und** **4****,** begrenzt, zwischen denen ein (geradliniger) Strömungskanal verläuft. Der Lüftungskanalabschnitt 20 kann aus metallischen oder polymeren Komponenten aufgebaut sein und beispielsweise einen quadratischen oder rechteckigen Querschnitt aufweisen. Die Querschnittsform und Dimensionen der Vorrichtung sind zumindest an den Enden des Lüftungskanalabschnitts 20 sind so konstruiert, dass die Vorrichtung in kommerzielle neue und bestehende Anlagen, die von verschiedenen Anlagenbauern angeboten werden, eingebaut werden kann.

In Richtung des zu reinigenden Gasstroms sind in einer Vorrichtung gemäß einer Ausführungsform der Erfindung ein Filter, das Strahlermodul, optional ein Ozonierungsraum und abschließend ein Aktivkohlefilter angeordnet.

Das Strahlermodul kann aus einer oder mehreren Strahlereinheiten bestehen, die ferner in mehreren Ebenen senkrecht zum Gasstrom angeordnet sein können.

Je nach gewünschter Behandlungsart, Photolyse, photo-initiiert, photokatalytisch oder kombiniert, wird die Art der Strahlungsquellen mit geeignetem Emissionsspektrum ausgewählt. Bei der photokatalytischen Behandlung werden Katalysatoroberflächen in geeigneter Anordnung zu den Strahlereinheiten des Strahlermoduls in der Vorrichtung angeordnet. Das mit den **Fig. 3** **und** **4****.** beschriebene Beispiel bezieht sich auf eine solche Vorrichtung, in der ein erfindungsgemäßes Strahlermodul zusammen mit Titandioxid-Katalysatorfiltern zur photokatalysierten Oxidation der organischen Verbindungen in dem Gasstrom verwendet wird.

Im Lüftungskanalabschnitt, der die Vorrichtung beherbergt, können eine Öffnung oder mehrere Öffnungen zur Wartung, Reparatur und/oder Austausch der Filter und Module bzw. deren Komponenten vorgesehen sein.

Der dem Strahlermodul vorgeschaltete Filter besteht aus einem Staubfilter, der je nach Anwendung dimensioniert wird. Vorteilhaft kann es sich dabei um einen Feinstaubfilter, z. B. Filterklasse F6 handeln, um nicht nur gröbere Partikel und Tröpfchen, sondern auch Feinstaub am Eintritt in die durch das Strahlermodul gebildete Reaktionszone zu hindern.

Das Strahlermodul, für das der Lüftungskanalabschnitt 20 in **Fig. 4** mit einem die Revisionsöffnung verschließenden Deckel 23 dargestellt ist, besteht aus jeweils drei in einer Ebene angeordneten Strahlereinheiten 1, die senkrecht zur Strömungsrichtung im Lüftungskanalabschnitt 20, abwechselnd zu Photokatalysatorschichten 24 angeordnet sind, gut in der Seitenansicht in **Fig. 3** zu erkennen. Vorzugsweise wird jede aus Strahlereinheiten 1 des Strahlermoduls gebildete Ebene beidseitig einer Photokatalysatorschicht 24 begrenzt. Anders als in **Fig. 3** **und** **4** dargestellt, können auch mehr oder weniger Photokatalysatorschichten und Strahlerebenen eingesetzt werden sowie die Anzahl der Strahlereinheiten 1 in einer Ebene variiert werden. Abhängig von der Schadstoffart, der Schadstoffbeladung und der Durchflussmenge können mehr oder weniger alternierend angeordnete Photokatalysatorschichten 24 und Strahlerebenen vorgesehen sein, wobei die erste und die letzte Schicht immer durch eine Photokatalysatorschicht 24 gebildet werden.

Für die Photokatalysator-Schichten 24 wird als Photokatalysator TiO₂ eingesetzt, das auf Trägermaterial chemisch fixiert, d. h. kovalent gebunden ist. Als Trägermaterial werden Quarz- oder Glasfasern oder Glas- oder Silikagelpartikel mit unterschiedlicher Porosität eingesetzt. Das mit der TiO₂-Beschichtung funktionalisierte Trägermaterial wird als Packung durch Netze 25, vorzugsweise Stahlnetze 25 rechtwinklig zum Gasfluss stabilisiert. Die Packung soll dabei einen Druckverlust von ca. 500 Pa nicht überschreiten.

Die Wirkungsweise des TiO₂-Photokatalysators wurde in der Literatur ausreichend beschrieben und ist dem Fachmann bekannt.

Im vorliegenden Beispiel weist jede Strahlerebene drei Strahlereinheiten 1 auf; die Anzahl der in einer Vorrichtung verwendeten Strahlereinheiten 1 pro Ebene hängt von den Abmessungen der Strahlereinheit 1 selbst und des Querschnitts des Lüftungskanalabschnitts ab. Die Strahlereinheiten 1 einer Strahlerebene sind rechtwinklig zum Gasfluss installiert. Bei einem rechtwinkligen oder quadratischen Strömungsquerschnitt werden die Strahlereinheiten 1 parallel zueinander angeordnet. Bei mehr als einer Strahlerebene können die Strahlereinheiten 1 benachbarter Strahlerebenen ebenfalls parallel zueinander oder gegebenenfalls um 90° versetzt angeordnet sein.

Da es bei zu hohen Ozonkonzentrationen zur Selbstentzündung an der Katalysatorschicht kommen kann, können als Strahlenquellen in den Strahlereinheiten zwischen den Photokatalysatorschichten UV-Strahler gewählt werden, die kein Ozon erzeugen, d. h. oberhalb 200 nm emittieren.

Beim Einsatz von VUV-Strahlung in Normalatmosphäre lässt sich die Bildung von Ozon nicht verhindern. Die Ozonbildung ist erwünscht und der Effekt des Ozons auf die Effizienz der Anlage kann durch die zusätzliche Zuführung von Ozon aus einem Ozonisator noch erhöht werden.

So können etwa in einer (figurativ nicht dargestellten) den Photokatalysatorschichten nachgeordneten Strahlerebene Ozon bildende UV-Strahler eingesetzt werden, da hier die auf die letzte Photokatalysatorschicht auftreffende Strahlung genutzt werden kann, das gebildete Ozon aber mit der Gasströmung abgeführt wird und nicht an die Photokatalysatorschicht gelangen kann.

Grundsätzlich ist aber auch der Einsatz von Strahlenquellen, die sowohl im VUV als auch UV-Bereich emittieren, zwischen den Photokatalysatorschichten möglich, da so die Effizienz der Anlage durch das gebildete Ozon und die damit geförderte Oxidationsreaktion weiter verbessert werden kann.

In einer Ausführungsform der Vorrichtung, in der sowohl die 254 nm Emission einer Strahlungsquelle zur Photokatalyse als auch die 185 nm Emission zur Photolyse von z. B. Sauerstoff genutzt werden, kann die Distanz zwischen den einzelnen Strahlereinheiten 1 so gewählt werden, dass sie maximal der doppelten Eindringtiefe der bei 185 nm emittierten Photonen in Luft entspricht. Die Distanz der endständigen Strahlereinheiten 1 zur Kanalinnenwand entspricht maximal der Eindringtiefe der bei 185 nm emittierten Photonen in Luft. Abhängig von der Dimension der modularen Abluftreinigungsvorrichtung ergibt sich damit die Anzahl der in einer Strahlerebene auf einer Linie eingesetzten Strahlereinheiten 1.

Die Distanz zwischen einer Strahlerebene und den vor- und nachgeordneten Photokatalysator-Schichten 24 wird so gewählt, dass sich eine möglichst homogene Verteilung der auf die Photokatalysator-Schicht 24 auftreffenden Strahlungsenergie ergibt.

Vorzugsweise werden Strahlungsquellen 2 mit einem Emissionsspektrum eingesetzt, das den UV-Spektralbereich von 100 bis 380 nm umfasst, insbesondere den UVC- und VUV-Spektralbereich. Werden Hg-Strahler ausgewählt, so sind die Emissionslinien von 185 bis 380 nm wesentlich. Emittierte Photonen aller dieser Linien sind im Prinzip geeignet den Photokatalysator elektronisch anzuregen.

Als Strahlungsquellen eignen sich vor allem Hg-Niederdruckstrahler mit und ohne Amalgam, die in dem synthetischen Quarzrohr 3, das für UVC- und VUV-Strahlung transparent ist, montiert sind. Die in der erfindungsgemäßen Reinigungsvorrichtung zum Einsatz kommenden Hg-Amalgam-Strahlungsquellen weisen stärkste Emissionswellenlängen bei 185 und 254 nm auf. Während mit der Emissionswellenlänge bei 254 nm praktisch aller eingesetzten Strahlungsquellen der TiO₂-Photokatalysator und Schadstoffe, die im UVC-Bereich absorbieren, angeregt werden können, wird die zusätzliche Emissionswellenlänge im VUV-Spektralbereich (185 nm) hauptsächlich durch den in der Normalatmosphäre enthaltenen molekularen Sauerstoff absorbiert.

Theoretisch wäre es auch möglich andere Strahlungsquellen einzusetzen, die im Spektralbereich 100 bis 380 nm emittieren. Doch scheitert dies in den meisten Fällen an der hohen IR-Emission oder an der ungenügenden Strahlungsenergie dieser Quellen.

In einer Ausführungsform der Erfindung besteht die Abluftreinigungsvorrichtung lediglich aus Filter, Strahlermodul mit oder ohne Photokatalysator und Aktivkohlefilter.

Ohne Photokatalysator können Strahlungsquellen eingesetzt werden, die mit der emittierten UVC- und VUV- Strahlung den oxidativen Abbau über direkte Photolyse der Schadstoffe und mittels des durch VUV aus Luftsauerstoff erzeugten Ozons bewirkt.

Wird ein Photokatalysator mit dem Strahlermodul eingesetzt, so kann an dieser Stelle durch Verwendung von Strahlereinheiten ohne VUV-Emission, sei es durch geeignete Wahl der Strahlungsquelle oder durch Filter, die Bildung von Ozon und die damit verbundene Herabsetzung des Zündpunkts eines explosionsfähigen Gemischs an der TiO₂-Katalysatorschicht verhindert werden.

Liegen in der zu reinigenden Abluft, bzw. dem Abgas hohe Konzentrationen organischer Schadstoffe vor und/oder bedarf es hoher Mineralisierungsraten, kann der Photokatalyse, durchgeführt mit dem Strahlermodul und den Photokatalysatorschichten, ein Ozonisierungsraum nachgeschaltet werden, in dem die in der Photokatalyse initiierte Oxidationsreaktion durch Zufluss von Ozon, das von einer externen Quelle stammt, z. B. einem extern angebrachten Ozonisator generiert wird, unterstützt wird. Das Ozon kann beispielsweise über einen aus einem perforierten, vertikal zum Gasfluss installierten ringförmigen Glasrohr in den Ozonierungsraum eingetragen werden. Der Ozonierungsraum wird der Photokatalyse nachgeschaltet, damit grössere Konzentrationen Ozon den Zündpunkt des Gasgemischs an der bestrahlten TiO₂-Packung nicht herabsetzen und damit eine Selbstentzündung auslösen können.

In diesem Fall besteht die Abluftreinigungsvorrichtung aus Filter, Strahlermodul mit Photokatalysatorschichten, dem Ozonierungsraum und dem Aktivkohlefilter.

Das Volumen des Ozonierungsraums kann den jeweiligen Bedürfnissen angepasst werden. Dazu kann das Strahlermodul innerhalb der durch den Lüftungskanalabschnitt begrenzten Reinigungsvorrichtung weiter vorne oder weiter hinten angeordnet werden.

Der die Reinigungsvorrichtung abschließende Aktivkohlefilter dient dazu, Oxidationsprodukte, die gasförmig oder als Aerosol anfallen, zu adsorbieren und nicht-reagiertes Ozon abzubauen. Liegen höhere Konzentrationen von nicht-reagiertem Ozon vor, kann ein Filter aus Aktivkohle mit aufgebrachtem Silber eingesetzt werden, wodurch der Ozonabbau beschleunigt und verbessert wird, so dass der die Reinigungsvorrichtung verlassende Luftstrom den Anforderungen bezüglich des maximal zulässigen Ozongehalts entspricht.

Je nach Bedarf können in einer Abluft- Abgas- oder Raumluftreinigungsanlage die photokatalytischen und photooxidativen Prozessstufen in Serie und/oder parallel eingesetzt werden.

Ferner umfasst die Abluftreinigungsvorrichtung eine Schalt- und Kontrollstelle, mit der die Abluftreinigungsvorrichtung, bzw. das Vorschaltgerät (die Vorschaltgeräte) der Strahlereinheiten des Strahlermoduls, das Steuergerät der Schutzgasüberdruckkapselung und die optionale zusätzliche Ozonzufuhr betrieben bzw. gesteuert oder geregelt wird.

Ferner kann die erfindungsgemäße Reinigungsvorrichtung, die z. B. als Raumluftreinigungsvorrichtung ausgeführt ist, Sensoren zur (redundanten) Messung der Ablufttemperatur und Konzentration des Rest-Ozons (z. B. zur Einhaltung des MAK-Wertes bzw. den Grenzwerten der TA Luft) aufweisen, wobei die erhaltenen Daten bei Überschreitung der vorgegebenen Grenzwerte zur Abschaltung von Strahlungsquellen und Ozonisator führen.

Mit einer erfindungsgemäßen Reinigungsvorrichtung wird die Effizienz der oxidativen Aufbereitung von Abgasen, Abluft und Raumluft erhöht bzw. optimiert, einerseits durch die erfindungsgemäße Anordnung der Strahler in Schichten zwischen Photokatalysatorschichten und dem nachgeschalteten Ozonierungsraum und andererseits indem Hg-Amalgam-Strahlungsquellen eingesetzt werden, die nicht nur zur oxidativen Photokatalyse und zur UVC-Photolyse von Schadstoffen, sondern zusätzlich auch zur VUV-Photolyse von Schadstoffen, zur Oxidantienbildung und infolge dessen zur Ozonierung der Schadstoffe und deren Oxidationsprodukte eingesetzt werden können.

Die Dimensionen des quadratischen (oder rechteckigen) Querschnitts lassen sich innerhalb der Grenzen der verfügbaren Strahlungsquellen an neue und bestehende Anlagen anpassen, und die Zahl und räumliche Anordnung der Strahlungsquellen ist variabel.

Die erfindungsgemäße Vorrichtung eignet sich sowohl zur Behandlung von Abgasen, die z. T. auch mit hohen Konzentrationen an organischen Verbindungen belastet sein können, als auch zur Behandlung von Abluft und Raumluft, mit den Aufgaben, toxische Mikroorganismen (Bakterien, Viren, Sporen) und Verbindungen durch Oxidation abzubauen und/oder unangenehme Geruchsbildung abzubauen.

Der Aktivkohlefilter ist in all jenen Fällen notwendig, wo durch die Bestrahlungsquellen oder durch Einsatz eines Ozonisators Ozon gebildet wird. Die Effizienz der Ozonvernichtung am Aktivkohlefilter kann durch Aufbringen von kolloidalem Silber auf die Aktivkohle erhöht werden.

## Patentansprüche

1. Strahlermodul, geeignet zur Verwendung in einer Abluft-, Abgas- oder Raumluftreinigungsvorrichtung, mit zumindest einer Strahlereinheit (1), die eine koaxial in einem Quarzglas-Hüllrohr (3) angeordnete Strahlungsquelle (2) aufweist,
**dadurch gekennzeichnet, dass**
- die Strahlereinheit (1) ein Kopfteil (10) aufweist, das mit dem Quarzglas-Hüllrohr (3) abdichtend und federnd gelagert verbunden ist und durch das sich eine Schutzgaseinleitungsvorrichtung (6') in einen Raum zwischen dem Hüllrohr (3) und der Strahlungsquelle (2) erstreckt,
- das Strahlermodul eine Schutzgasüberdruckeinrichtung aufweist, die über die Schutzgaseinleitungsvorrichtung (6') in dem Raum zwischen dem Quarzglas-Hüllrohr (3) und der Strahlungsquelle (2) eine Schutzgasatmosphäre bereitstellt, die einen Überdruck gegenüber dem Umgebungsdruck von zumindest 0,8 mbar aufweist.

2. Strahlermodul nach Anspruch 1,
**dadurch gekennzeichnet, dass**
- sich von der Schutzgaseinleitungsvorrichtung (6') ein Kapillarrohr (6) zwischen dem Hüllrohr (3) und der Strahlungsquelle (2) bis zum entgegengesetzt liegenden Ende des Hüllrohrs (3) erstreckt und dort einen Auslass für das Schutzgas in den Raum zwischen dem Hüllrohr (3) und der Strahlungsquelle (2) bereitstellt, und
- in dem Kopfteil (10) eine Schutzgasausleitungsvorrichtung (6") vorgesehen ist, und
- die Schutzgasüberdruckeinrichtung eine mit der Einleitungsvorrichtung (6') verbundene Schutzgaszuleitung und eine mit der Ausleitungsvorrichtung (6") verbundene Schutzgasableitung aufweist, in der eine Druckmessvorrichtung vorgesehen ist, die mit einem Steuergerät verbunden ist, das ein in der Schutzgaszuleitung angeordnetes Magnetventil, das bevorzugt ein Proportionalventil ist, steuert.

3. Strahlermodul nach Anspruch 2,
**dadurch gekennzeichnet, dass**
das Strahlermodul mehrere Strahlereinheiten (1) umfasst, wobei die Schutzgaszuleitung über die Schutzgaseinleitungsvorrichtung (6') und Schutzgasausleitungsvorrichtung (6") der Strahlereinheiten (1) parallel oder in Reihe mit der Schutzgasableitung verbunden ist.

4. Strahlermodul nach Anspruch 2 oder 3,
**dadurch gekennzeichnet, dass**
das Steuergerät mit einem die Strahlungsquelle (2) mit Strom versorgendem Vorschaltgerät zum Abschalten der Strahlungsquelle (2) bei einem durch die Druckmessvorrichtung festgestellten Druck unterhalb des Mindestüberdrucks von 0,8 mbar und oberhalb eines vorab festgelegten zulässigen Maximalüberdrucks gekoppelt ist.

5. Strahlermodul nach zumindest einem der Ansprüche 1 bis 4,
**dadurch gekennzeichnet, dass**
das Kopfteil (10) mittels eines Ringflansches (4) mit dem Hüllrohr (3) verbunden ist, wobei
- der Ringflansch (4) bevorzugt mit einer konischen Innenfläche ausgebildet ist, die mit dem konisch ausgebildeten offenen Ende des Hüllrohrs (3) korrespondiert, wobei sich der Konus zu der von dem Kopfteil (10) weg weisenden Seite verjüngt, und/oder
- an der Innenfläche des Ringflansches (4) zumindest zwei umlaufende Nuten zur Aufnahme von Dichtringen (5) vorgesehen sind, und/oder
- der Ringflansch (4) zur Befestigung der Strahlereinheit (1) in oder an einer Abluft-, Abgas- oder Raumluftreinigungsvorrichtung ausgebildet ist, und/oder
- das Kopfteil (10) becherartig ausgebildet ist und einen nach außen weisenden Kragen (12) aufweist, mit dem das Kopfteil (10) an dem Hüllrohr (3) anliegt, wobei die durch den Kragen (12) gebildete Stirnfläche des Kopfteils (10) eine Ringnut zur Aufnahme eines Dichtrings (13) aufweist, und/oder
- der Ringflansch (4) einen zum Kopfteil (10) weisenden ringförmigen Überstand (15) aufweist, der zumindest teilweise in eine zweite auf der Stirnfläche des Kragens (12) angeordnete Ringnut (14) hineinragt, und/oder
- die federgelagerte Verbindung des Kopfteils (10) mit dem Hüllrohr (3) über eine an dem Kragen (12) anliegende Scheibe (17), die über Federschrauben (16) mit dem Ringflansch (4) verbunden ist, bereitgestellt wird.

6. Strahlermodul nach einem der Ansprüche 1 bis 5,
**dadurch gekennzeichnet, dass**
das Quarzglas-Hüllrohr (3) ein Hüllrohr (3) aus synthetischem Quarzglas ist und eine Wandstärke von zumindest 3 mm aufweist und zumindest einmal getempert und 10 bar druckgeprüft ist.

7. Abluft-, Abgas- oder Raumluftreinigungsvorrichtung mit einem Abgas- oder Lüftungskanalabschnitt (20), das einen Einlass (21) und einen Auslass (22) für einen zu reinigenden Abluft-, Abgas- oder Raumluftstrom aufweist,
**dadurch gekennzeichnet, dass**
die Vorrichtung ein Strahlermodul nach zumindest einem der Ansprüche 1 bis 6 zur photooxidativen und/oder photokatalytischen Behandlung des zu reinigenden Abluft-, Abgas- oder Raumluftstroms aufweist.

8. Abluft-, Abgas- oder Raumluftreinigungsvorrichtung nach Anspruch 7, **dadurch gekennzeichnet, dass**
die Vorrichtung stromaufwärts des Strahlermoduls einen Filter und/oder stromabwärts des Strahlermoduls einen Ozonierungsraum und/oder stromabwärts des Strahlermoduls, bevorzugt stromabwärts des Ozonierungsraums einen Aktivkohlefilter aufweist,
wobei die Vorrichtung eine Ozon-Zufuhrvorrichtung aufweist, die mit einer Ozonquelle verbunden ist und die bevorzugt ein perforiertes, senkrecht zur Strömungsrichtung in dem Ozonierungsraum angeordnetes ringförmiges Glasrohr aufweist, wobei
eine Größe des Ozonierungsraums an eine Schadstoffbeladung des Abluft-, Abgas- oder Raumluftstroms durch Variation des Abstands zwischen dem Strahlermodul und dem Aktivkohlefilter anpassbar ist.

9. Abluft-, Abgas- oder Raumluftreinigungsvorrichtung nach Anspruch 7 oder 8,
**dadurch gekennzeichnet, dass**
die Vorrichtung stromabwärts des Ozonierungsraums, bevorzugt stromabwärts des Aktivkohlefilters
- einen Ozonsensor, der zum Abschalten der Strahlungsquelle (2) und/oder der Ozon-Zufuhrvorrichtung bei einer durch den Ozonsensor festgestellten Ozonkonzentration oberhalb eines vorgegebenen Grenzwerts mit dem Vorschaltgerät der Strahlungsquelle (2) und/oder der Ozon-Zufuhrvorrichtung gekoppelt ist,
und/oder
- einen Temperatursensor aufweist, der zum Abschalten der Strahlungsquelle (2) bei einer durch den Temperatursensor festgestellten Temperatur des Abluft-, Abgas- oder Raumluftstroms oberhalb eines vorgegebenen Grenzwerts mit dem Vorschaltgerät der Strahlungsquelle (2) gekoppelt ist.

10. Abluft-, Abgas- oder Raumluftreinigungsvorrichtung nach zumindest einem der Ansprüche 7 bis 9,
**dadurch gekennzeichnet, dass**
die Strahlereinheiten (1) des Strahlermoduls in zumindest einer Ebene senkrecht zur Strömungsrichtung des Abluft-, Abgas- oder Raumluftstrom angeordnet sind, wobei beidseitig davon senkrecht zur Strömungsrichtung Photokatalysatorschichten (24) angeordnet sind, die Trägerkörper aus Quarz- oder Glasfasern oder Glas- oder Silikagelpartikel mit einer Titandioxid-Beschichtung aufweisen, wobei jede Photokatalysatorschicht (24) durch ein Paar von Netzstrukturen (25) mit dem Innenquerschnitt des Lüftungskanalabschnitts (20) entsprechenden Abmessungen begrenzt wird, und eine Packung der Trägerkörper zwischen den Netzstrukturen (25) einen Druckverlust von maximal 500 Pa aufweist.
